# EUROPEAN PATENT APPLICATION

(11) **EP 3 243 506 A1**
(43) Date of publication of application: **15.11.2017**
(21) Application number: 16461520.5
(22) Date of filing: 09.05.2016
(51) Int. Cl.: A61K 9/16, A61K 9/20, A61K 31/00

(54) **PHARMACEUTICAL COMPOSITION**

(71) Applicant: Adamed sp. z o.o., 05-152 Czosnów k/Warszawy (PL)
(72) Inventor: Cieplucha, Agnieszka, 05-825 Grodzisk Mazowiecki (PL)
(74) Representative: Sulikowski, Daniel

(57) **Abstract**

The present invention relates to a pharmaceutical composition comprising rosuvastatin and ezetimibe for oral administration in the form of a blend of a first component comprising rosuvastatin or a pharmaceutically acceptable salt thereof, wherein the component is dry-granulated, a second component comprising ezetimibe or a pharmaceutically acceptable salt thereof, wherein the component is wet-granulated, and an optionally extragranular phase component, characterized in that the pharmaceutical composition is free from any rosuvastatin stabilizers, and d₉₀ value for granules of the first component is 800 µm or less. The invention relates also to an uncoated tablet comprising the composition, and a method of manufacturing the composition.

## Description

### Field of the invention

The present invention relates to a pharmaceutical composition comprising rosuvastatin and ezetimibe for oral administration comprising dry-granulated component comprising rosuvastatin or a pharmaceutically acceptable salt thereof and wet-granulated component comprising ezetimibe or a pharmaceutically acceptable salt thereof.

### Prior art

Simplicity in industrial scale manufacturing is a key factor that allows saving considerable financial outlays. Many technologies involve equipment that is expensive to purchase, use expensive substances, often harmful and toxic, and provide unsatisfactory yields of the final forms. It is the most important, therefore in general, to provide a pharmaceutical composition that both has a high chemical stability and fulfils therapeutic criteria, and can be obtained in a simple method using conventional means, basic pharmaceutical technologies while greatly simplifying production and reducing its costs.

Compositions comprising rosuvastatin and ezetimibe in one dosage form are known in the art. For example, compositions comprising rosuvastatin stabilized with a rosuvastatin stabilizer are disclosed in EP2448919, EP2566465, EP2844233, WO2015044698, WO2015093859, WO2015044698, and WO2009024889.

Specifically, WO2009024889 discloses a monolayer tablet comprising dry-granulated rosuvastatin component and wet-granulated ezetimibe component, wherein the rosuvastatin component contains rosuvastatin stabilizer. The tablet is coated with a coating comprising ferric oxide. No mention is made about stability and release kinetics of the active ingredients.

Furthermore, WO2015102400 discloses monolayer tablet comprising rosuvastatin and ezetimibe, wherein the ezetimibe part is wet-granulated and rosuvastatin part is a powder mixture. All examples given in the description encompass rosuvastatin stabilized with a rosuvastatin stabilizer. According to comparative examples, lack of rosuvastatin stabilizer leads to considerable degradation of rosuvastatin.

On the other hand, WO2015044698 relates to a pharmaceutical composition containing rosuvastatin and ezetimibe wherein the active ingredients are present in spatially separated or contacting physical phases and wherein the active ingredient release from the two phases takes place in temporarily separated manner rather than occurring at the same time or begins delayed from one phase compared to the other. According to the description, without spatial separation of rosuvastatin and ezetimibe phases, like in the case of monolayer and bilayer tablet, rosuvastatin release affects ezetimibe release kinetics in such way that that the rosuvastatin salt decreases the dissolution of ezetimibe from the tablet in such an extent that the thus obtained combined composition fails to satisfy therapeutic criteria. As an example, a capsule containing separate rosuvastatin tablet and ezetimibe tablet is given. The application is silent about stability of active ingredients in such a dosage form. The rosuvastatin tablet contains no rosuvastatin stabilizer.

WO2013066279 discloses bilayer tablet comprising rosuvastatin and ezetimibe. The information is given about neither rosuvastatin stabilizer nor stability of such a composition.

There is only one product commercially available comprising both rosuvastatin and ezetimibe. Rosulip Plus (Egis) is in the form of hard gelatin capsule containing separate mini-tablets of rosuvastatin zinc and ezetimibe. The mini-tablets are free from a rosuvastatin stabilizer. The capsule contains iron oxide.

Thus, there is still a need in the art, on one hand, for a pharmaceutical composition comprising rosuvastatin and ezetimibe that can be obtained in a simple way, without need for using any rosuvastatin stabilizers, using conventional means and basic pharmaceutical technologies, wherein the composition can be presented in the form of a monolayer tablet or a capsule, and on the other hand, for a pharmaceutical composition that has a high chemical stability and fulfills therapeutic criteria.

The above requirements are met by the pharmaceutical composition of the present invention.

### Definitions

The term "rosuvastatin stabilizer" can be defined as a chemical compound known in the art to stabilize rosuvastatin, and includes, for example, polyvalent metal salts as disclosed, for example WO015466 and WO01546 including monobasic, dibasic and tribasic phosphates, carbonates and bicarbonates, oxides and hydroxides, aluminates and silicates of magnesium, calcium, aluminum, zinc, iron, as well as monovalent metal salts of the anions mentioned above of sodium, potassium, ammonium, and organic bases as, for example, amines like meglumine. The specific examples of rosuvastatin stabilizers are calcium hydrogenphosphate, calcium phosphate, magnesium oxide, magnesium hydroxide, and ferric oxide.

The term "unifoimity of mixing" relates to reaching of homogeneous mixture by mixing, i.e. a mixture which is characterized by substantially the same distribution of components in a given sample throughout the whole weight or volume of the mixture.

The term "granule size" has its meaning in the art, and relates to granules' particle size distribution estimated by analytical dry sieving method indicated in pharmaceutical guidelines, e.g. described in ICH Analytical Sieving General Chapter. Thus, the test method used was a sieving method utilizing at least 6 sieves with mesh size [mm]: 0.80, 0.50, 0.30, 0.20, 0.10, 0.05, and applying mechanical agitation for 10 minutes with the set values of amplitude: 1.5 and intervals: 10 sec.

The terms "d₉₀", "d₅₀" and "d₁₀" relate to particle size characteristics and follow the definitions given in 2.9.35. chapter of Ph. Eur. The terms characterize the cumulative distribution of particles - powders (e.g. active substances, estimated by the relevant method, e.g. Malvern Mastersizer) or granules (e.g. RSV granulates, estimated by analytical sieving, e.g. sieve analysis).

### Detailed Description of the Invention

The present invention relates to a pharmaceutical composition comprising rosuvastatin and ezetimibe for oral administration in the form of a blend of:
a) a first component comprising rosuvastatin or a pharmaceutically acceptable salt thereof, wherein the component is dry-granulated,
b) a second component comprising ezetimibe or a pharmaceutically acceptable salt thereof, wherein the component is wet-granulated,
c) an optionally extragranular phase component,
characterized in that
the pharmaceutical composition is free from any rosuvastatin stabilizer, and d₉₀ value for granules of the first component is less than 800 µm or less.

The inventors found that lack of any rosuvastatin stabilizer in the composition of the invention and the presence of the first component in a dry-granulated form positively influences stability of the whole composition in relation to both rosuvastatin and ezetimibe by decreasing impurity profiles as shown in Examples in comparison to other compositions comprising stabilizers and/or rosuvastatin in other form than dry-granulated.

Furthermore, it turned out that by taking granules comprising rosuvastatin of d₉₀ value above 800 µm, release of rosuvastatin is significantly slowed. Therefore, granule sizes below 800 µm are important. Additional advantage of such a granule size is that it allows sufficient uniformity of mixing in the pharmaceutical composition of the invention.

The dry-granulated first component can be obtained by direct dry-granulation of the powder mixture comprising rosuvastatin or a pharmaceutically acceptable salt thereof and suitable excipients selected from fillers, disintegrants, binders, and lubricants. Other excipient can be also included, if needed. Suitable excipients used in pharmacy can be found in "Handbook of Pharmaceutical Excipients" edited by R. C. Rowe, P. J. Sheskey, and S. Owen, Pharmaceutical Press, edition VII.

The particle size of rosuvastatin or a salt thereof used may be d₉₀ less than about 60 µm, more particularly less than about 30 µm. The particle size of ezetimibe or a salt thereof used may be d₉₀ less than about 10 µm, more particularly less than about 8 µm.

The rosuvastatin or a pharmaceutically acceptable salt thereof can be rosuvastatin calcium.

The dry-granulation method used to obtain the first component can be selected from roller compaction and slugging. The methods are described, for example, in "Pharmaceutical Manufacturing Handbook" edited by Shayne Cox Gad, published by John Wiley & Sons, Inc., Hoboken, New Jersey.

The ezetimibe or a pharmaceutically acceptable salt thereof can be ezetimibe.

The wet-granulated second component can be obtained by a suitable granulation method known in the art using powder mixtures of suitable excipients as mentioned above. Other excipient can be also included, if needed.

The wet-granulation method used to obtain the second component can be selected from can be selected from low shear, high shear, and fluid bed granulation.

Preferably, rosuvastatin or a pharmaceutically acceptable salt thereof is rosuvastatin calcium, and ezetimibe or a pharmaceutically acceptable salt thereof is ezetimibe.

The first component comprising rosuvastatin or a pharmaceutically acceptable salt thereof preferably consists of rosuvastatin or a pharmaceutically acceptable salt thereof, at least one filler, at least one disintegrant, and/or at least one binder. The excipients can be selected from the groups as indicated below.

The second component comprising ezetimibe or a pharmaceutically acceptable salt thereof preferably consists of ezetimibe or a pharmaceutically acceptable salt thereof, at least one filler, at least one disintegrant, at least one binder and/or at least one surfactant. The excipients can be selected from the groups as indicated below.

The extragranular phase component, if present, consists preferably of excipients selected from at least one filler, at least one binder, at least one disintegrant, at least one surfactant, and/or at least one lubricant.

The at least one filler can be selected independently form anhydrous or monohydrate lactose, sucrose, microcrystalline cellulose, starch, pregelatinized starch, microcrystalline cellulose coated with colloidal silica. Preferably, the at least one filler is selected from lactose monohydrate, microcrystalline cellulose, or a mixture thereof.

The at least one binder can be selected independently from hydroxypropylcellulose, hydroxypropylmethylcellulose, methylcellulose, carboxymethylcellulose, hydroxycellulose, hydroxyethylcellulose, polyvinyl alcohol, polivinylpyrrolidone. Preferably, the at least one binder is selected from hydroxypropyl cellulose, polyvinylpyrrolidone or a mixture thereof.

The at least one disintegrant can be selected independently from carboxymethylcellulose sodium salt, sodium croscarmellose, microcrystalline cellulose, crospovidone, sodium starch glycolate. Preferably, the at least one disintegrant is selected from sodium carboxymethyl cellulose, crospovidone or a mixture thereof.

The at least one surfactant can be selected independently form sodium laurylsulfate, polysorbate 80, polyoxyethylene sorbiten. Preferably, the at least one surfactant is sodium laurylsulfate.

The at least one lubricant can be selected independently from magnesium stearate, calcium stearate, sodium stearyl fumarate, hydrogenated vegetable oil, for example, hydrogenated castor oil. Preferably, the at least one lubricant is magnesium stearate.

Preferably, the pharmaceutical composition of the invention consists of:
40-80%wt. of the first component comprising rosuvastatin or a pharmaceutically acceptable salt thereof,
15-25%wt. of the second component comprising ezetimibe or a pharmaceutically acceptable salt thereof
0-40%wt. of the extragranular phase component,
wherein the weight percentages add up to 100%.

Specifically, it was observed that less than 40%wt. of the first component in the pharmaceutical composition requires high concentration of active substance in it. This affects rosuvastatin release and a delay in the release compared to the reference product kinetics is observed. On the other hand, more than 80%wt. of the first component provides the first component having a low concentration of active substance in it, and this translates into faster release compared to the reference product kinetics. As a result, range 40-80%wt. of the first component turned out to be advantageous. The same behavior was observed for ezetimibe. Namely, less than 15%wt. of the second component gives a delay in releasing of ezetimibe, whereas more than 25%wt. gives too fast release of the active ingredient.

Such a percentage composition gives particularly suitable pharmaceutical composition in terms of fulfilling therapeutic criteria. This means that the release of both rosuvastatin and ezetimibe from the pharmaceutical composition is bioequivalent to the single referent compositions comprising rosuvastatin and ezetimibe, respectively.

Preferably, in the pharmaceutical composition of the invention, d₉₀ for granules of the first component is 500 µm or less. When d₉₀ value for granules of the first component is 500 µm or less, particularly good uniformity of mixing is achieved.

Another aspect of the invention provides an uncoated tablet comprising the pharmaceutical composition. It is the further advantage of the present invention that the pharmaceutical composition can be directly compressed into a tablet without need for carrying out subsequent steps of coating. In the pharmaceutical composition of the invention, the lack of coating additionally improves the chemical stability since there is no wet (water) process, and with the dry-granulated first component offers very efficient masking of an unpleasant bitter taste of rosuvastatin. Furthermore, lack of coating strongly facilitates the economical aspect of production procedure by elimination of additional coating step, further resulting in saving production time and avoiding additional costs connected with reagents and coating equipment.

Another aspect of the invention provides a method for obtaining the pharmaceutical composition; the method comprises the following steps:
a) preparation of the first component comprising rosuvastatin or a pharmaceutically acceptable salt thereof
   I) blend rosuvastatin or a pharmaceutically acceptable salt thereof with all excipients except lubricant to homogeneity,
   II) add lubricant and blend,
   III) dry-granulate by roller-compaction or slugging,
   IV) break-down the resulting slugs or sheets using the suitable milling technique to produce granules,
b) preparation of the second component comprising ezetimibe or a pharmaceutically acceptable salt thereof
   I) mix ezetimibe or a pharmaceutically acceptable salt thereof with at least one filler, at least one disintegrant and/or at least surfactant in a high-shear granulator to homogeneity,
   II) granulate mixture of I) with a binder solution in water,
   III) unify granule size by sieving or screening,
c) preparation of blend for tableting/capsuling
   I) blend granulates from a) and b) and optional extragranular phase component to homogeneity.

Blending operations can be performed in a suitable blender, preferably in a container blender.

The first component comprising rosuvastatin or a salt thereof is prepared in the present invention using dry granulation technology.

In the dry methods of granulation, the primary powder blend particles are aggregated under high pressure using one of two main dry granulation processes: either roller-compaction or slugging. In both cases the intermediate products, slugs or sheets, are broken down using a suitable milling technique to produce granular material, which can be additionally sieved to separate the desired size fraction.

Such a way of processing rosuvastatin powder blend is very advantageous for several reasons. This method allows elimination of water that affects rosuvastatin stability, and obtaining a granulate having grain size that is optimal for further processing (uniform mixing with the second component comprising ezetimibe or a salt thereof and optional extragranular phase component without the effect of physical segregation). Furthermore, the system of rosuvastatin and excipients included in the granule is mechanically stable and allows significant separation of the granules from the potential environmental effects of other substances (also present in the composition, e.g., introduced together with the second component) which on one hand seals and stabilizes rosuvastatin in fixed microenvironment without need for any rosuvastatin stabilizer, and on the other hand significantly reduces or even completely eliminates an unpleasant bitter taste of rosuvastatin, which can be very advantageous in the point of view of uncoated tablet. Additionally, the proposed route of rosuvastatin component manufacturing enables incorporating broad ratio (% wt) of this active substance into dry granulate intermediate, not available for other dry pharmaceutical processes (e.g. direct compression).

Ezetimibe is subjected to a process of wet granulation (low shear, high shear or in a fluidized bed), to improve significantly its solubility and to achieve the desired in vitro and in vivo release profile. The resulting granulate is dried and screened to obtain a proper distribution of the particles, allowing durable and uniform mixing with the first component and additional excipients.

The resulting components are blended together, and depending on the desired final form, compressed into tablet or filled into capsules.

As mentioned above, preferably, the tablet is an uncoated tablet.

Alternatively, the tablet can be also coated with any suitable coating, e.g. functional coating, when targeted release is desirable. Such coatings can be found in "Pharmaceutical Manufacturing Handbook" edited by Shayne Cox Gad, published by John Wiley & Sons, Inc., Hoboken, New Jersey.

The method of the invention enables producing the pharmaceutical composition using conventional tools and well-known pharmaceutical technologies, while significantly simplifying the manufacturing process, and reducing its costs comparing to manufacturing standards known in the art.

### EXAMPLES

### Example 1. Preparation of pharmaceutical composition of the invention

Exemplary pharmaceutical compositions of the invention are presented in the table below.

| **Composition ingredients** | | **Composition No.** | | | | | |
|---|---|---|---|---|---|---|---|
| | | 1 | 2 | 3 | 4 | 5 | 6 |
| | | **Amount of ingredient / dosage [mg]** | | | | | |
| | Ezetimibe | 10,0 | 10,0 | 10,0 | 10,0 | 10,0 | 10,0 |
| | Lactose monohydrate | 49,0 | 25,0 | 49,0 | 39,0 | 29,0 | 18,0 |
| ezetimibe granulate (EZE GRA) | Microcrystalline cellulose | 10,0 | 21,5 | 9,0 | 20,0 | 10,0 | 9,5 |
| | Sodium croscarmellose | 4,0 | 2,0 | 4,0 | 1,0 | 4,0 | 2,0 |
| | Sodium laurylsulfate | 2,0 | 1,5 | 2,0 | 3,0 | 2,0 | 1,5 |
| | HPC | - | - | 4,0 | - | - | 4,0 |
| | Povidone | 4,0 | 4,0 | - | 6,0 | 4,0 | - |
| *EZE GRA mg* | | *79,0* | *64,0* | *78,0* | *79,0* | *59, 0* | *45, 0* |
| *EZE GRA* % *l composition* | | *18*,*8* | *20,0* | *23,3* | *24,7* | *15,1* | *25, 0* |
| | Rosuvastatin calcium | 20,8 | 20,8 | 10,4 | 10,4 | 5,2 | 5,2 |
| rosuvastatin granulate (RSV GRA) | Lactose monohydrate | 159,3 | 50,0 | 229,3 | 120,0 | 194,9 | - |
| | Microcrystalline cellulose | 105,3 | 50,0 | - | 25,0 | 85,4 | 95,0 |
| | Crospovidone | 12,0 | 8,5 | 14,0 | 7,6 | 12,0 | 5,0 |
| | Magnesium stearate | 2,6 | 1,2 | 3,3 | 1,2 | 2,5 | 1,2 |
| *RSV GRA mg* | | *300, 0* | *130,5* | *257,0* | *164, 2* | *300, 0* | *106,4* |
| *RSV GRA %* / *composition* | | *71,4* | *40,8* | *76,7* | *51,3* | *76,9* | *59,1* |
| Extragranular phase component | Microcrystalline cellulose | 24,2 | 114,1 | - | 58,8 | 14,2 | 14,2 |
| | Sodium starch glycolate | - | 8,2 | - | 15,0 | 12,6 | - |
| | Sodium croscarmellose | 12,6 | - | - | - | - | 12,0 |
| | Magnesium stearate | 4,2 | 3,2 | - | 3,0 | 4,2 | 2,4 |
| *EXTRAGRANULAR PHASE (E.P.), mg* | | *41,0* | *125,5* | *0,0* | *76,8* | *31,0* | *28,6* |
| *E.P. %* /*composition* | | *9,8* | *39,2* | *0,0* | *24,0* | *8, 00* | *15,9* |
| TOTAL: | | 420,0 | 320,0 | 335,0 | 320,0 | 390,0 | 180,0 |

The pharmaceutical compositions are obtained using following technological process.
1. Preparation of the ezetimibe granulate:
   1.1. Ezetimibe, lactose monohydrate, microcrystalline cellulose, sodium lauryl sulfate, and sodium croscarmellose are mixed in a high shear granulator to homogeneity.
   1.2. A binder solution is prepared by dissolution of indicated binder (povidone or HPC) in water.
   1.3. The mixture of item 1.1 is granulated with the solution of item 1.2.
   1.4. The granulate of item 1.3. is dried, and then unified by screening through a sieve 0.5-0.8 mm.
2. Preparation of the rosuvastatin granulate:
   2.1. Rosuvastatin calcium, lactose monohydrate, microcrystalline cellulose, crospovidone are blend in a container blender to homogeneity.
   2.2. The mixture of item 2.1. is blended with the lubricant (magnesium stearate).
   2.3. The mixture of item 2.2. is compacted using a roller compactor.
   2.4. The sheets from 2.3 are broken down using the suitable milling technique to produce granules
3. Preparation of a final blend of the pharmaceutical composition
   3.1. Ezetimibe granulate of item 1.4, rosuvastatin granulate of item 2.3, and, if present, extragranular phase component except lubricant are blended in a container blender to homogeneity.
   3.2 If present, lubricant is added to item of 3.1. and blended,

The final blend of item 3.1 or 3.2 is compressed into a tablet on a rotary tableting machine equipped with the suitable tools to obtain the desired shape of the tablet (e.g., round or oval, with optional engraving). The tablet is not subjected to any coating processes.

In another embodiment, hard gelatine capsules No. 0 were filled with the blend obtained in item 3.1 or 3.2.

### Example 2. Stability studies

Medicines must comply with the requirements of the chemical purity immediately after preparation (comply with the limits indicated in the release specification of the drug product), and within the period set by the relevant guidelines in time and storage conditions (comply with the limits indicated in the shelf-life specification of the drug product).

According to Ph. Eur., the main impurities being the degradation products of rosuvastatin are:
1) Impurity B-RSV (Anti-isomer), with the chemical name (3RS,5RS,6E)-7-[4-(4-fluorophenyl)-6-(1-methylethyl)-2-[methyl(methylsulfonyl)amino]-pyrimidin-5-yl]-3,5-dihydroxyhept-6-enoic acid,
2) Impurity C-RSV (5-ketoacid), with the chemical name (3R,6E)-7-[4-(4-fhiorophenyl)-6-(1-methylethyl)-2-[methyl(methylsulfonyl)amino]pyrimidin-5-yl]-3-hydroxy-5-oxohept-6-enoic acid, and
3) Impurity D-RSV (Lactone), with the chemical name N-(4-(4-fluorophenyl)-5-[(E)-2-[(2S,4R)-4-hydroxy-6-oxotetrahydro-2H-pyran-2-yl]ethenyl]-6-(1-methylethyl)-pyrimidin-2-yl)-N-methylmethanesulfonamide

The third, lactone derivative formed by acidic hydrolysis referred to as an impurity D (IMP D-RSV), is the most relevant in the pharmaceutical art according to the available literature and the internal studies. Therefore, the level of IMP D-RSV became a basic measure of the stability of a pharmaceutical composition comprising rosuvastatin. Limit for the content of IMP D RSV is max. 0.5% at the end of the aging period (indicated in shelf-life specification), either analyzed after 6 months' testing at the accelerated storage conditions, or based on the real time data available from the long term storage.

Numerical data presented in these examples therefore relate mainly to the level of IMP D RSV, although routine chemical purity analysis always included a full profile of known and unknown impurities as indicated above.

The pharmaceutical compositions obtained in Example 1 in the form of uncoated tablets were subjected to aging at elevated temperature 40°C and 75 % RH humidity, i.e., under accelerated condition. Table 1 shows results of chemical purity after storage under indicated conditions for exemplary compositions of the invention (composition no. 1, and no. 5), and comparative compositions (composition no. 1', and no. 5') having the same qualitative and quantitative composition, obtained using the same method, but without compacting step of a mixture of rosuvastatin with excipients (mixture of the powder mixture containing rosuvastatin and the second wet-granulated component compressed into uncoated tablet).

**Table 1. The drug product's chemical stability under accelerated conditions - for pharmaceutical compositions of the invention and comparative compositions.**

| *Composition No.* | | *1* | *5* | *1'* | *5'* |
|---|---|---|---|---|---|
| ezetimibe/rosuvastatin [mg]: | | 10/20 | 10/5 | 10/20 | 10/5 |
| RSV dry granulation: | | Yes | Yes | No | No |
| IMP D RSV [%] | start | ND | ND | ND | ND |
| | 1 month | <0.10 | 0.17 | 0.44 | 0.69 |
| requirement: ≤0.5% after 6 months | 3 months | 0.15 | 0.30 | NT | NT |
| | 6 months | 0.22 | 0.42 | NT | NT |

| | | | | | |
|---|---|---|---|---|---|
| ND - not detected; NT - not tested; compositions 1' and 5' are comparative and obtained as in Example 1, but without dry-granulation step of the first component comprising rosuvastatin. | | | | | |

As is clear from the table above, the use of dry-granulation technology for the first component significantly improves the stability of the pharmaceutical composition of the invention. Rosuvastatin impurity D levels for compositions without dry-granulation (i.e., roller-compacting) were already close to or higher than 0.5% after 1 month of storage (according to the relevant stability guidelines the limit shall not be exceeded within 6 months of storage in these conditions; although results from accelerated testing studies are not always predictive for real time data physical changes, for the subject EZE-RSV combination the accelerated stability data were confirmed to be very supportive and compatible with the impurity profile obtained at the long term storage condition).

In addition, for comparative compositions no. 1' and no. 5', a significant levels of the other known and unknown rosuvastatin degradation products were reported. Thus, dry-granulation of the first component comprising rosuvastatin is an essential step that ensures stability of the rosuvastatin component.

Further, influence of the presence of rosuvastatin stabilizer was investigated. Toward this end, further comparative compositions no. 6'- no. 10' were prepared.

**Table 2. Comparative compositions comprising rosuvastatin stabilizers.**

| **Composition ingredients** | | **Composition No.** | | | | |
|---|---|---|---|---|---|---|
| | | 6' | 7' | 8' | 9' | 10' |
| | | **amount of ingredient / dosage [mg]** | | | | |
| | Ezetimibe | 10,0 | 10,0 | 10,0 | 10,0 | 10,0 |
| | Lactose monohydrate | 49,0 | 49,0 | 49,0 | 49,0 | 68,2 |
| Ezetimibe granulate (EZE GRA) | Microcrystalline cellulose | 10,0 | 10,0 | 10,0 | 10,0 | - |
| | Sodium croscarmellose | 4,0 | 4,0 | 4,0 | 4,0 | 3,0 |
| | Sodium laurylsulfate | 2,0 | 2,0 | 2,0 | 2,0 | 2,0 |
| | Povidone | 4,0 | 4,0 | 4,0 | 4,0 | 3,0 |
| | Rosuvastatin calcium | 20,8 | 5,2 | 5,2 | 20,8 | 10,4 |
| | Lactose monohydrate | 159,3 | 39,8 | 39,8 | 159,3 | 108,6 |
| Rosuvastatin granulate (RSV GRA)* | Microcrystalline cellulose | 100,2 | 21,1 | 21,1 | 100,2 | 8,0 |
| | Magnesium oxide | 5,2 | 5,2 | 5,2 | - | - |
| | Magnesium hydroxide | - | - | - | 5,2 | - |
| | Dibasic calcium phosphate | - | - | | - | 7,5 |
| | Crospovidone | 12,0 | 3,0 | 3,0 | 12,0 | 3,0 |
| | Magnesium stearate | 2,6 | 0,6 | 0,6 | 2,6 | 0,8 |
| Extragranular phase compoment | Microcrystalline cellulose | 24,2 | 6,1 | 6,1 | 24,2 | 18,0 |
| | Sodium croscarmellose | 12,6 | 3,2 | 3,2 | 12,6 | 3,0 |
| | Magnesium stearate | 4,2 | 1,1 | 1,1 | 4,2 | 1,5 |
| | Crospovidone | - | - | - | - | 3,0 |
| TOTAL: | | 420,00 | 164,3 | 164,3 | 420,00 | 250,00 |

| | | | | | | |
|---|---|---|---|---|---|---|
| * Comparative compositions 6', 7', 9' were obtained as in Example 1 in the form of uncoated tablet, comparative composition 8' - composition as obtained in Example 1 in the form of uncoated tablet but without compacting the first component comprising rosuvastatin; 10' - composition according to WO2009024889, Example 4, without coating. | | | | | | |

The comparative compositions np. 6' - no. 10' were subjected again to aging at elevated temperature 40°C and 75 % RH humidity, i.e., under accelerated condition. The results are presented in the table below.

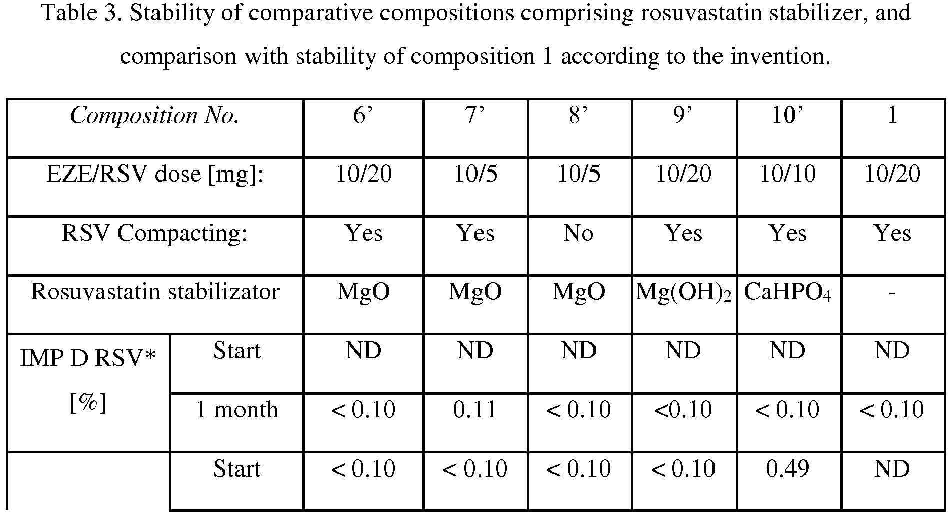

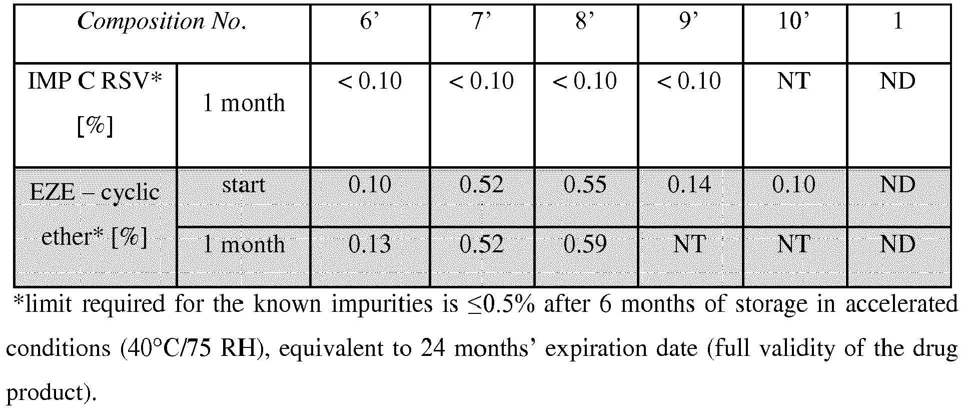

It turned out that although rosuvastatin impurity D did not appear at elevated levels in comparative compositions no. 6'- no. 10', either rosuvastatin impurity C was present at significant level in the composition stabilized with CaHPO₄ (comparative composition no. 10'), or ezetimibe, comprised in the second component, underwent significant degradation to ezetimibe cyclic ether impurity (comparative compositions no. 6'- no. 9'). This degradation process was particularly marked in case of low dose of rosuvastatin. As analyzed, the chemical purity of ezetimibe in composition without compacting of rosuvastatin was even lower (8'), and this supports additionally importance of compacting step for successful manufacturing of composition comprising both ezetimibe and rosuvastatin.

In case of composition of the invention, the impurities were not detected or detected only at very low level even after 6 months of storage under accelerated conditions. Thus, the absence of rosuvastatin stabilizer does not only mean that one of one of excipients can be eliminated, but this simply improves the stability.

Finally, stability of exemplary composition of the invention was compared with a stability of the only available market product Rosulip Plus. The comparison was performed again under accelerated conditions. The results are presented in the Table 4 below.

**Table 4. Comparison of a composition of the invention with commercially available product.**

| *Product* | | *Rosulip Plus* | *Composition no. 1* |
|---|---|---|---|
| EZE/RSV dose [mg]: | | 10/20 | 10/20 |
| IMP D RSV [%] | start | 0.50 | ND |
| | 1 month | 0.94 | <0.10 |
| requirement: ≤0.5% after 6 months | 2 months | 1.14 | 0.15 |

Accordingly, composition of the invention exhibits highly improved stability comparing to commercially available product.

### Example 3. Dissolution studies

To characterize the dissolution behavior of the drug product, the following general specification valid for immediate release pharmaceutical compositions should be fulfilled: at least 80 %wt. of each active ingredient is dissolved from the formulation within 30 minutes.

As the bio-relevant conditions the dissolution media presently recommended for the individual active substances were chosen, e.g., 0.45% SLS in 0.05 M Acetate Buffer solution of pH 4.5 - for ezetimibe dissolution evaluation, or 0.05 M Sodium Citrate Buffer solution of pH 6.6 - for rosuvastatin dissolution evaluation (according FDA Dissolution Methods Database). Of course, alternative dissolution methods properly justified and correlated with in vivo drugs' behavior may be applied to verify dissolving characteristic of the pharmaceutical composition of the invention.

All compositions of the invention met the above requirement, and in each case more than 80%wt. of the relevant active ingredient dissolved within 30 min.

### Example 4. Bioequivalence

As mentioned above, pharmaceutical compositions of the invention are particularly suitable in terms of fulfilling therapeutic criteria. This means that the release of both rosuvastatin and ezetimibe from the pharmaceutical composition is bioequivalent to the single referent compositions comprising ezetimibe and rosuvastatin, respectively.

Following the general recommendations described in the relevant guidelines e.g. Guideline on the investigation of bioequivalence, Doc. Ref.: CPMP/EWP/QWP/1401/98 Rev. 1/Corr, the assessment of bioequivalence based upon 90% confidence intervals for the ratio of the population geometric means (test/reference) for the parameters under consideration, namely Cₘₐₓ and AUC, should be contained within the acceptance interval of 80.00-125.00%. As confirmed in vivo, for the uncoated tablets comprising pharmaceutical composition according to the present invention, the bioequivalent criteria were easily met.

## Claims

1. A pharmaceutical composition comprising rosuvastatin and ezetimibe for oral administration in the form of a blend of:
a) a first component comprising rosuvastatin or a pharmaceutically acceptable salt thereof, wherein the component is dry-granulated,
b) a second component comprising ezetimibe or a pharmaceutically acceptable salt thereof, wherein the component is wet-granulated,
c) an optionally extragranular phase component,
**characterized in that**
the pharmaceutical composition is free from any rosuvastatin stabilizers, and d₉₀ value for granules of the first component is 800 µm or less.

2. The pharmaceutical composition of claim 1 **characterized in that** the pharmaceutical composition consists of:
40-80%wt. of the first component comprising rosuvastatin or a pharmaceutically acceptable salt thereof,
15-25%wt. of the second component comprising ezetimibe or a pharmaceutically acceptable salt thereof
0-40%wt. of the extragranular phase component,
wherein the weight percentages add up to 100%.

3. The pharmaceutical composition of claim 1 - 2 **characterized in that** d₉₀ value for granules of the first component is 500 µm or less.

4. The pharmaceutical composition of claim 1-3 **characterized in that** rosuvastatin or a pharmaceutically acceptable salt thereof is rosuvastatin calcium, and ezetimibe or a pharmaceutically acceptable salt thereof is ezetimibe.

5. The pharmaceutical composition of claim 1-4 **characterized in that** the first component comprising rosuvastatin or a pharmaceutically acceptable salt thereof consists of rosuvastatin or a pharmaceutically acceptable salt thereof, at least one filler, at least one disintegrant, at least one binder, and/or at least one lubricant.

6. The pharmaceutical composition of claim 1-5 **characterized in that** the second component comprising ezetimibe or a pharmaceutically acceptable salt thereof consists of ezetimibe or a pharmaceutically acceptable salt thereof, at least one filler, at least one disintegrant, at least one binder and/or at least one surfactant.

7. The pharmaceutical composition of claim 1-6 **characterized in that** the extragranular phase component consists of excipients selected form at least one filler, at least one binder, at least one disintegrant, at least one surfactant, and/or at least one lubricant.

8. The pharmaceutical composition of claim 5-7 **characterized in that** at least one filler is selected from lactose monohydrate, microcrystalline cellulose, or a mixture thereof

9. The pharmaceutical composition of claim 5-7 **characterized in that** at least one binder is selected from hydroxypropyl cellulose, polyvinylpyrrolidone or a mixture thereof.

10. The pharmaceutical composition of claim 5-7 **characterized in that** at least one disintegrant is selected from sodium carboxymethyl cellulose, crospovidone or a mixture thereof.

11. An uncoated tablet comprising a pharmaceutical composition as described in claims 1-10.

12. Method for obtaining the pharmaceutical composition as defined in any of claims 1-10 **characterized in that** it comprises the following steps:
a) preparation of the first component comprising rosuvastatin or a pharmaceutically acceptable salt thereof
I) blend rosuvastatin or a pharmaceutically acceptable salt thereof with all excipients except lubricant to homogeneity,
II) add lubricant,
III) dry-granulate by roller-compaction or slugging,
IV) break-down the resulting slugs or sheets using the suitable milling technique to produce granules,
b) preparation of the second component comprising ezetimibe or a pharmaceutically acceptable salt thereof
I) mix ezetimibe or a pharmaceutically acceptable salt thereof with at least one filler, at least one disintegrant and/or at least surfactant in a high-shear granulator to homogeneity,
II) granulate mixture of I) with a binder solution in water,
III) unify granule size by sieving or screening (0.5-0.8 mm),
c) preparation of blend for tableting/capsuling
I) blend granulates from a) and b) and optional extragranular phase to homogeneity.
